# EUROPÄISCHE PATENTSCHRIFT

(11) **EP 1 105 365 B1**
(45) Veröffentlichungstag und Bekanntmachung des Hinweises auf die Patenterteilung: **19.06.2002**
(21) Anmeldenummer: 99940184.7
(22) Anmeldetag: 10.08.1999
(51) Int. Cl.: C07C 45/33, C07C 45/35, C07C 47/22, C07C 51/215, C07C 51/25, C07C 57/04

(54) **VERFAHREN ZUR HERSTELLUNG VON ACROLEIN UND/ODER ACRYLSÄURE AUS PROPAN**
METHOD FOR PRODUCING ACROLEIN AND/OR ACRYLIC ACID FROM PROPANE
PROCEDE DE PRODUCTION D'ACROLEINE ET/OU D'ACIDE ACRYLIQUE A PARTIR DE PROPANE

(30) Priorität: 19.08.1998 DE 19837519
(43) Veröffentlichungstag der Anmeldung: 13.06.2001
(73) Patentinhaber: BASF AKTIENGESELLSCHAFT, 67056 Ludwigshafen (DE)
(72) Erfinder: MACHHAMMER, Otto, D-68163 Mannheim (DE); TENTEN, Andreas, D-67487 Maikammer (DE); JACHOW, Harald, D-64625 Bensheim (DE); HAUPT, Susanne, D-63069 Offenbach (DE); ARNOLD, Heiko, D-68159 Mannheim (DE); UNVERRICHT, Signe, D-68169 Mannheim (DE)
(86) Internationale Anmeldenummer: EP9905792
(87) Internationale Veröffentlichungsnummer: WO0010957

(56) Entgegenhaltungen:
- EP-A- 0 117 146
- WO-A-97/36849
- GB-A- 2 118 939

## Beschreibung

Die vorliegende Erfindung betrifft ein Verfahren zur Herstellung von Acrolein und/oder Acrylsäure aus Propan, bei dem man das Propan in einer ersten Reaktionszone einer partiellen homogenen und/oder heterogen katalysierten Oxidehydrierung mit molekularem Sauerstoff zu Propen unterwirft und anschließend das in der ersten Reaktionszone gebildete, Propen enthaltende, Produktgasgemisch ohne Abtrennung eines Produktgasgemischbestandteils in wenigstens eine weitere Reaktionszone führt und in dieser wenigstens einen weiteren Reaktionszone das im Produktgasgemisch der ersten Reaktionszone enthaltene Propen in Begleitung aller Produktgasgemischbestandteile der ersten Reaktionszone einer gasphasenkatalytischen Oxidation zu Acrolein und/oder Acrylsäure unterwirft, aus dem Produktgasgemisch der gasphasenkatalytischen Oxidation darin enthaltenes Acrolein und/oder Acrylsäure sowie Wasser abtrennt und das im dabei verbleibenden Restgasstrom enthaltene nicht umgesetzte Propan als Bestandteil des Restgasstromes in die erste Reaktionszone zurückführt.

Acrolein und Acrylsäure sind bedeutende Zwischenprodukte, die beispielsweise im Rahmen der Herstellung von Wirkstoffen und Polymerisaten Verwendung finden.

Das gegenwärtig großtechnisch zur Herstellung von Acrolein und/oder Acrylsäure überwiegend angewandte Verfahren bildet die gasphasenkatalytische Oxidation von Propen (z.B. EP-A 575 897), wobei das Propen überwiegend als Nebenprodukt der Ethylenherstellung durch Steamcracken von Naphtha erzeugt wird.

Da die sonstigen Anwendungsgebiete des Propens, z.B. die Herstellung von Polypropylen, sich immer weiter ausdehnen, wäre es vorteilhaft, über ein großtechnisch anwendbares, wettbewerbsfähiges Verfahren zur Herstellung von Acrolein und/oder Acrylsäure zu verfügen, dessen Rohstoffbasis nicht Propen, sondern z.B. das als Erdgasbestandteil reichlich natürlich vorkommende Propan ist.

Aus der US-A 3 798 283 ist bekannt, daß Propan im Beisein von molekularem Sauerstoff bei erhöhter Temperatur homogen zu Propen oxidehydriert werden kann. Als Sauerstoffquelle kommen dabei sowohl reiner Sauerstoff als auch Gemische aus Sauerstoff und Inertgas in Betracht.

Aus der DE-A 20 58 054 und der DE-A 1 95 30 454 ist bekannt, daß die Oxydehydrierung von Propan zu Propen auch heterogen katalysiert durchgeführt werden kann.

Die US-A 3 161 670, die EP-A 117 446 und die DE-A 33 13 573 betreffen Verfahren zur Herstellung von Acrolein und/oder Acrylsäure, bei denen zunächst Propan durch heterogen katalysierte Dehydrierung unter Sauerstoffausschluß zu Propen dehydriert wird.

Danach wird das Propen enthaltende Produktgemisch einer heterogen katalysierten Gasphasenoxidation unterworfen. Nachteilig an dieser Verfahrensweise ist jedoch, daß der für die nicht oxidative Dehydrierung des Propans benötigte Katalysator durch Kohlenstoffablagerungen relativ rasch deaktiviert wird und daher häufig regeneriert werden muß. Ein weiterer Nachteil dieser Verfahrensweise ist die mit der nicht oxidativen Propandehydrierung einhergehende Wasserstoffbildung.

Die DE-A 33 13 573 erwähnt zwar die prinzipielle Möglichkeit einer Kopplung von oxidativer Dehydrierung von Propan zu Propen mit nachfolgender heterogen katalysierter Propenoxidation. Sie enthält jedoch keine weitergehenden Angaben zur Durchführung eines solchen Verfahrens.

Die EP-A 293 224, das US-A 5 198 578 und das US-A 5 183 936 lehren, daß ein erhöhter N₂-Anteil im Verdünnungsgas der katalytischen Gasphasenoxidation von Propen zu Acrolein und/oder Acrylsäure von Nachteil ist. Die EP-A 293 224 regt ferner an, die oxidative Dehydrierung von Propan zu Propen und die katalytische Gasphasenoxidation von Propen zum Zweck der Herstellung von Acrolein und/oder Acrylsäure miteinander zu kombinieren.

In Catalysis Today 13, 1992, S. 673 bis 678 kombinieren Moro-oka et al eine homogene oxidative Dehydrierung von Propan zu Propen mit einer nachfolgenden heterogen katalysierten Oxidation des Dehydrierproduktgemisches zu Acrolein und/oder Acrylsäure. Die entsprechende Verfahrenskombination empfehlen Moro-oka et al in Applied Catalysis, 70 (2), 1991, S. 175 bis 187. Der Empfehlung der EP-A 293 224, der US-A 5 198 578 und der US-A 5 183 936 entsprechend verwenden Moro-oka et al in allen Fällen als Sauerstoffquelle für die Oxidehydrierstufe entweder reinen molekularen Sauerstoff oder an Stickstoff entreicherte Luft. Ebenso verfahren wird in der CN-A 1105352.

Die WO 97/36849 schließt zwar eine unmittelbare Verwendung von Luft als Sauerstoffquelle für eine katalytische oxidative Dehydrierung von Propan zu Propen im Rahmen einer Kopplung mit einer nachfolgenden Propenoxidation zu Acrolein und/oder Acrylsäure nicht aus, zur Begrenzung des N₂-Gehaltes in der anschließenden Propenoxidationsstufe sieht sie aber nur die Möglichkeit, in der Oxidehydrierstufe als Sauerstoffquelle bereits ein Gasgemisch einzusetzen, das wenigstens 90 mol-% Sauerstoff enthält. Dies gilt insbesondere unter Berücksichtigung einer Rückführung des nicht umgesetztes Propan enthaltenden, von Acrolein und/oder Acrylsäure befreiten, Reaktionsgasgemisches der katalytischen Gasphasenoxidationsstufe in die Oxidehydrierstufe, würde eine solche Rückführung doch ein Aufpegeln des Stickstoffgehaltes in der Gasphasenoxidation bedingen. Darüber hinaus sieht die WO 97/36849 für den Fall einer kontinuierlichen Verfahrensweise mit Kreisgasführung zur Unterdrückung einer unerwünschten Aufpegelung nachteiliger Reaktionsgasgemischbestandteile lediglich einen Auslaß (purge) an Kreisgas und keine Komponentenabtrennung vom Kreisgas vor.

Aus Wirtschaftlichkeitsgründen kommt für großtechnische Gasphasenoxidationen als Ausgangsmaterial für die molekulare Sauerstoffquelle im wesentlichen nur Luft in Betracht.

Vor diesem Hintergrund sind die vorgenannten Verfahrensweisen insofern von Nachteil, als infolge der Ähnlichkeit von O₂ und N₂ ausgehend von Luft die alleinige Maßnahme einer Vorab- Stickstoff-/Sauerstofftrennung zur Herstellung von reinem Sauerstoff bzw. einer an Stickstoff entreicherten Luft zur Beschränkung des Stickstoffgehaltes bei einer nachfolgenden Oxidation des im Dehydrierproduktgemisch enthaltenen Propens sehr energieaufwendig ist.

Aufgabe der vorliegenden Erfindung war daher ein Verfahren zur Herstellung von Acrolein und/oder Acrylsäure aus Propan, bei dem man das Propan in einer ersten Reaktionszone einer partiellen homogenen und/oder heterogen katalysierten Oxidehydrierung mit molekularem Sauerstoff zu Propen unterwirft und anschließend das in der ersten Reaktionszone gebildete, Propen enthaltende, Produktgasgemisch ohne Abtrennung eines Produktgasgemischbestandteils in wenigstens eine weitere Reaktionszone führt und in dieser wenigstens einen weiteren Reaktionszone das im Produktgasgemisch der ersten Reaktionszone enthaltene Propen in Begleitung aller Produktgasgemischbestandteile der ersten Reaktionszone einer gasphasenkatalytischen Oxidation zu Acrolein und/oder Acrylsäure unterwirft, aus dem Produktgasgemisch der gasphasenkatalytischen Oxidation darin enthaltenes Acrolein und/oder Acrylsäure sowie Wasser abtrennt, und das im dabei verbleibenden Restgasstrom enthaltene nicht umgesetzte Propan als Bestandteil des Restgasstromes in die erste Reaktionszone zurückführt, zur Verfügung zu stellen, bei dem die Beschränkung des Stickstoffgehalts in der Propenoxidationsstufe in einer weniger energieaufwendigen Weise als im Stand der Technik erfolgt.

Demgemäß wurde ein Verfahren zur Herstellung von Acrolein und/oder Acrylsäure aus Propan, bei dem man das Propan in einer ersten Reaktionszone einer partiellen homogenen und/oder heterogen katalysierten Oxidehydrierung mit molekularem Sauerstoff zu Propen unterwirft und anschließend das in der Reaktionszone gebildete, Propen enthaltende, Produktgasgemisch ohne Abtrennung eines Produktgasgemischbestandteils in wenigstens eine weitere Reaktionszone führt und in dieser wenigstens einen weiteren Reaktionszone das im Produktgasgemisch der ersten Reaktionszone enthaltene Propen in Begleitung aller Produktgasgemischbestandteile der ersten Reaktionszone einer gasphasenkatalytischen Oxidation zu Acrolein und/oder Acrylsäure unterwirft, aus dem Produktgasgemisch der gasphasenkatalytischen Oxidation darin enthaltenes Acrolein und/oder Acrylsäure sowie Wasser abtrennt und das im dabei verbleibenden Restgasstrom enthaltene nicht umgesetzte Propan als Bestandteil des Restgasstroms in die erste Reaktionszone zurückführt, dadurch gekennzeichnet, daß man dem der ersten Reaktionszone zugeführten Reaktionsgasausgangsgemisch den in der ersten Reaktionszone benötigten, von Kreissauerstoff verschiedenen, molekularen Sauerstoff in Form von Luft zusetzt und vor der Rückführung des Restgasstromes in die erste Reaktionszone wenigstens einen Teil des im Restgasstrom enthaltenen Luftstickstoff vom Restgasstrom abtrennt.

Die partielle Vorteilhaftigkeit der erfindungsgemäßen Verfahrensweise gegenüber den nächstliegenden Verfahren des Standes der Technik liegt als Ergebnis eingehender Forschung darin begründet, daß insbesondere die im Rahmen der oxidativen Dehydrierung des Propans unter Verwendung einer Stickstoff enthaltenden Sauerstoffquelle erfolgende chemische Bindung des molekularen Sauerstoff implizit einen Teil der zur Trennung von Luftstickstoff und Luftsauerstoff zu investierenden Trennarbeit leistet. Entsprechendes gilt für die nachfolgenden Oxidationsstufen. Die Verschiedenheit zwischen den dabei entstehenden, Sauerstoff enthaltenden, polaren Verbindungen (z.B. Acrylsäure, Acrolein, H₂O, CO, CO₂) und N₂ ist wesentlich ausgeprägter als die Verschiedenheit zwischen N₂ und O₂, weshalb ihre Trennung von Stickstoff, im Unterschied zu einer Stickstoff-/Sauerstofftrennung, mit sehr viel geringerem Aufwand möglich ist. Das gleiche gilt für eine N₂-Abtrennung von den übrigen möglichen Komponenten des hauptsächlich aus N₂ und Propan bestehenden Restgasstromes, da sowohl die Verschiedenheit von Propan und N₂ als auch die Verschiedenheit des N₂ von den übrigen möglichen Komponenten des Restgasstromes sehr viel größer als die Verschiedenheit von O₂ und N₂ ist. In der Regel ist eine vergleichsweise wenig aufwendige rektifikative N₂-Abtrennung möglich.

Gestaltet man im Rahmen des erfindungsgemäßen Verfahrens die erste Reaktionszone als eine homogene Oxidehydrierung, so läßt sich diese prinzipiell z.B. wie in den Schriften US-A 3 798 283, CN-A 1 105 352, Applied Catalysis, 70 (2), 1991, S. 175 bis 187, Catalysis Today 13, 1992, S. 673 bis 678 und der älteren Anmeldung DE-A 1 96 22 331 beschrieben durchführen, sieht man davon ab, daß erfindungsgemäß als Sauerstoffquelle (abgesehen von Sauerstoffkreisgas) Luft einzusetzen ist.

Die Temperatur der homogenen Oxidehydrierung wird man zweckmäßig im Bereich von 300 bis 700°C, vorzugsweise im Bereich von 400 bis 600°C, besonders bevorzugt im Bereich von 400 bis 500°C liegend wählen. Der Arbeitsdruck kann 0,5x10⁵ - 1x10⁷ Pa (0,5 bis 100 bar) oder 1x10⁵ - 50x10⁵ Pa (1 bis 50 bar) betragen. Häufig wird er bei 1x10⁵ - 20x10⁵ Pa (1 bis 20 bar) oder bei 1x10⁵ - 10x10⁵ Pa (1 bis 10 bar) liegen.

Die Verweilzeit des Reaktionsgasgemisches unter Oxidehydrierbedingungen liegt üblicherweise bei 0,1 bzw. 0,5 bis 20 sec, vorzugsweise bei 0,1 bzw. 0,5 bis 5 sec. Die Realisierung der homogenen Oxidehydrierung kann z.B. in einem separaten Reaktor erfolgen. Als Reaktor kann dann z.B. ein Rohrofen oder ein Rohrbündelreaktor verwendet werden, wie z.B. ein Gegenstromrohrofen mit Rauchgas als Wärmeträger, oder ein Rohrbündelreaktor mit Salzschmelze als Wärmeträger.

Das Propan zu Sauerstoff Verhältnis im für eine homogene Oxidehydrierung einzusetzenden Reaktionsgasausgangsgemisch kann 0,5 : 1 bis 40 : 1 betragen. Erfindungsgemäß von Vorteil ist, wenn das Molverhältnis von Propan zu molekularem Sauerstoff im Reaktionsgasausgangsgemisch ≤ 6 : 1 bzw. ≤ 5 : 1 beträgt. In der Regel wird vorgenanntes Verhältnis ≥ 1 : 1 bzw. ≥ 2 : 1 betragen. Der Stickstoffanteil im Reaktionsgasausgangsgemisch ist in der Regel Konsequenz der vorgenannten Forderung, da das Reaktionsgasausgangsgemisch neben Propan und Luft normalerweise im wesentlichen keine weiteren Gase umfaßt. Selbstverständlich kann das Reaktionsgasausgangsgemisch aber auch weitere, im wesentlichen inerte, Bestandteile wie H₂O, CO₂, CO, N₂, Edelgase und/oder Propen umfassen. Propen als Bestandteil des Reaktionsgasausgangsgemisches ist beispielsweise dann gegeben, wenn als Ausgangspropan die aus der Raffinerie kommende C₃-Fraktion oder die C₃-Fraktion aus den Leichtkohlenwasserstoffen des Ölfelds verwendet wird, die einen Anteil an Propen von bis zu 10 Gew.-% aufweisen können. Ferner kann es von der erfindungsgemäß erforderlichen Kreisgasrückführung herrühren. In die Oxidehydrierung rückgeführte Komponenten werden in dieser Schrift ganz allgemein als Kreisgas bezeichnet. Infolge Kreisgasrückführung kann z.B. der Stickstoffanteil im Reaktionsgasausgangsgemisch bis zu 60 mol-% oder bis zu 50 mol-% betragen. Die erfindungsgemäße Kreisgasrückführung kann auch dazu führen, daß das Reaktionsgasausgangsgemisch bei kontinuierlicher Verfahrensweise bis zu 5 mol-% an Gasen wie CO, CO₂, Ethen und H₂O aufweist. Günstig für eine homogene oxidative Dehydrierung von Propan zu Propen ist es, wenn das Verhältnis der Oberfläche des Reaktionsraumes zum Volumen des Reaktionsraumes möglichst klein ist. Dies ist eine Konsequenz des radikalischen Mechanismus der homogenen oxidativen Propandehydrierung, wirken Reaktionsraumoberflächen in der Regel doch als Radikalfänger. Besonders günstige Oberflächenmaterialien sind Aluminiumoxide, Quarzglas, Borosilicate, Edelstahl und Aluminium.

Gestaltet man im Rahmen des erfindungsgemäßen Verfahrens die erste Reaktionsstufe als eine heterogen katalysierte Oxidehydrierung, so läßt sich diese prinzipiell z.B. wie in den Schriften US-A 4,788,371, CN-A 1073893, Catalysis Letters 23 (1994) 103 - 106, W. Zhang, Gaodeng Xuexiao Xuebao, 14 (1993) 566, Z. Huang, Shiyou Huagong, 21 (1992) 592, WO 97/36849, DE-A 1 97 53 817, US-A 3 862 256, US-A 3 887 631, DE-A 1 95 30 454, US-A 4 341 664, J. of Catalysis 167, 560 - 569 (1997), J. of Catalysis 167, 550 - 559 (1997), Topics in Catalysis 3 (1996) 265 - 275, US-A 5 086 032, Catalysis Letters 10 (1991) 181 - 192, Ind. Eng. Chem. Res. 1996, 35, 14 - 18, US-A 4 255 284, Applied Catalysis A: General, 100 (1993) 111 - 130, J. of Catalysis 148, 56 - 67 (1994), V. Cortés Corberän and S. Vic Bellón (Editors), New Developments in Selective Oxidation II, 1994, Elsevier Science B.V., S. 305 - 313, 3rd World Congress on Oxidation Catalysis, R.K. Grasselli, S.T. Oyama, A.M. Gaffney and J.E. Lyons (Editors), 1997, Elsevier Science B.V., S. 375 ff beschrieben durchführen, sieht man davon ab, daß erfindungsgemäß als Sauerstoffquelle (abgesehen von Sauerstoffkreisgas) Luft einzusetzen ist. Insbesondere können alle in den vorgenannten Schriften genannten Oxidehydrierkatalysatoren eingesetzt werden. Das für die vorgenannten Schriften gesagte gilt auch für:
a) Otsuka, K.; Uragami, Y.; Komatsu, T.; Hatano, M. in Natural Gas Conversion, Stud. Surf. Sci. Catal.; Holmen A.; Jens; K.-J.; Kolboe, S., Eds.; Elsevier Science: Amsterdam, 1991; Vol. 61, p 15;
b) Seshan, K.; Swaan, H.M.; Smits, R.H.H.; van Ommen, J.G.; Ross, J.R.H. in New Developments in Selective Oxidation; Stud. Surf. Sci. Catal.; Centi, G.; Trifirò, F., Eds.; Elsevier Science: Amsterdam 1990; Vol. 55, p 505;
c) Smits, R.H.H.; Seshan, K.; Ross, J.R.H. in New Developments in Selective Oxidation by Heterogeneous Catalysis; Stud. Surf. Sci. Catal.; Ruiz, P.; Delmon, B., Eds.; Elsevier Science: Amsterdam, 1992 a; Vol. 72, p 221;
d) Smits, R.H.H.; Seshan, K.; Ross, J.R.H. Proceedings, Symposium on Catalytic Selective Oxidation, Washington DC; American Chemical Society: Washington, DC, 1992 b; 1121;
e) Mazzocchia, C.; Aboumrad, C.; Daigne, C.; Tempesti, E.; Herrmann, J.M.; Thomas, G. Catal. Lett. 1991, 10, 181;
f) Bellusi, G.; Conti, G.; Perathonar, S.; Trifirò, F. Proceedings, Symposium on Catalytic Selective Oxidation, Washington, DC; American Chemical Society: Washington, DC, 1992; p 1242;
g) Ind. Eng. Chem. Res. 1996, 35, 2137 - 2143 und
h) Symposium on Heterogeneons Hydrocarbon Oxidation Presented before the Division of Petroleum Chemistry, Inc. 211 th National Meeting, American Chemical Society New Orleans, LA, March 24 - 29, 1996.

Erfindungsgemäß besonders geeignete Oxidehydrierkatalysatoren sind die Multimetalloxidmassen bzw. -katalysatoren A der DE-A 1 97 53 817, wobei die in der vorgenannten Schrift als bevorzugt genannten Multimetalloxidmassen bzw. -katalysatoren A ganz besonders günstig sind. D.h., als Aktivmassen kommen insbesondere Multimetalloxidmassen der allgemeinen Formel I

M¹ ₐMo_{1-b}M² _{b}Oₓ (I),

mit
- M¹ =: Co, Ni, Mg, Zn, Mn und/oder Cu,
- M² =: W, V, Te, Nb, P, Cr, Fe, Sb, Ce, Sn und/oder La,
- a =: 0,5 bis 1,5,
- b =: 0 bis 0,5 sowie
- x =: eine Zahl, die durch die Wertigkeit und Häufigkeit der von Sauerstoff verschiedenen Elemente in I bestimmt wird,
in Betracht.

Prinzipiell können erfindungsgemäß geeignete Aktivmassen I in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 450 bis 1000°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, Sauerstoff und NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Amminkomplexsalze, Ammonium-Salze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu vollständig gasförmig entweichenden Verbindungen zerfallen und/oder zersetzt werden können, können in das innige Trockengemisch zusätzlich eingearbeitet werden). Das innige Vermischen der Ausgangsverbindungen zur Herstellung von Multimetalloxidmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wäßrigen Lösung und/oder Suspension miteinander vermischt. Besonders innige Trockengemische werden beim beschriebenen Trockenverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt. Besonders geeignete Ausgangsverbindungen des Mo, V, W und Nb sind deren Oxoverbindungen (Molybdate, Vanadate, Wolframate und Niobate) bzw. die von diesen abgeleiteten Säuren. Dies gilt insbesondere für die entsprechenden Ammoniumverbindungen (Ammoniummolybdat, Ammoniumvanadat, Ammoniumwolframat).

Die Multimetalloxidmassen I können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Geeignete Hohlzylindergeometrien sind z.B. 7 mm x 7 mm x 4 mm oder 5 mm x 3 mm x 2 mm oder 5 mm x 2 mm x 2 mm (jeweils Länge x Außendurchmesser x Innendurchmesser). Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie er z.B. aus der DE-A 2909671 oder aus der EP-A 293859 bekannt ist. Zweckmäßigerweise kann zur Beschichtung der Trägerkörper die aufzubringende Pulvermasse befeuchtet und nach dem Aufbringen, z.B. mittels heißer Luft, wieder getrocknet werden. Die Schichtdicke der auf den Trägerkörper aufgebrachten Pulvermasse wird zweckmäßigerweise im Bereich 50 bis 500 µm, bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silicate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt.

Die Reaktionstemperatur der heterogen katalysierten Oxidehydrierung des Propans wird man zweckmäßig im Bereich von 300 bis 600°C, häufig im Bereich von 350 bis 500°C liegend wählen. Als Arbeitsdruck werden 0,5x10⁵ - 10x10⁵ Pa (0,5 bis 10 bar) bzw. 1x10⁵ - 10x10⁵ Pa (1 bis 10 bar) oder 1x10⁵ - 5x10⁵ Pa (1 bis 5 bar) empfohlen. Arbeitsdrucke oberhalb von 1x10⁵ Pa (1 bar), z.B. bei 1,5x10⁵ - 10x10⁵ Pa (1,5 bis 10 bar), haben sich als vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie z.B. in der EP-A 700 893 und in der EP-A 700 714 sowie der in diesen Schriften zitierten Literatur beschrieben sind. Dies kann z.B. in einem separatem Reaktor erfolgen. Die mittlere Verweilzeit des Reaktionsgasgemisches in der Katalysatorschüttung liegt im Normalfall bei 0,5 bis 20 sec. Das Propan zu Sauerstoff Verhältnis im für die heterogen katalysierte Propanoxidehydrierung einzusetzenden Reaktionsgasausgangsgemisch kann erfindungsgemäß 0,5 : 1 bis 40 : 1 betragen. Erfindungsgemäß von Vorteil ist, wenn das Molverhältnis von Propan zu molekularem Sauerstoff im Reaktionsgasausgangsgemisch ≤ 6 : 1 bzw. ≤ 5 : 1 beträgt. In der Regel wird vorgenanntes Verhältnis ≥ 1 : 1 bzw. 2 : 1 betragen. Der Stickstoffanteil im Reaktionsgasausgangsgemisch ist in der Regel Konsequenz der vorgenannten Forderung, da das Reaktionsgasausgangsgemisch neben Propan und Luft normalerweise im wesentlichen keine weiteren Gase umfaßt. Selbstverständlich kann das Reaktionsgasausgangsgemisch aber auch weitere, im wesentlichen inerte, Bestandteile wie H₂O, CO₂, CO, N₂, Edelgase und/oder Propen umfassen. Propen als Bestandteil des Reaktionsgasausgangsgemisches ist beispielsweise dann gegeben, wenn als Ausgangspropan die aus der Raffinerie kommende C₃-Fraktion oder die C₃-Fraktion aus den Leichtkohlenwasserstoffen des Ölfelds verwendet wird, die einen Anteil an Propen von bis zu 10 Gew.-% aufweisen können. Ferner kann es Konsequenz der erfindungsgemäß erforderlichen Kreisgasrückführung sein. Infolge Kreisgasrückführung kann z.B. der Stickstoffanteil im Reaktionsgasausgangsgemisch der heterogen katalysierten Propanoxidehydrierung im Rahmen des erfindungsgemäßen Verfahrens bei kontinuierlicher Durchführung bis zu 60 mol-% oder bis zu 50 mol-% betragen. Die erfindungsgemäße Kreisgasrückführung kann auch dazu führen, daß das Reaktionsgasausgangsgemisch bei kontinuierlicher Verfahrensweise bis zu 5 mol-% Gase wie CO, CO₂, Ethan, Methan, Ethen und/oder H₂O aufweist.

Häufig wird sowohl der O₂-Umsatz der homogenen als auch der katalytischen oxidativen Propandehydrierung im Rahmen des erfindungsgemäßen Verfahrens (bei einfachem Durchgang) ≥ 50 mol.-% bzw. ≥70 mol.-% betragen. Selbstredend können homogene und katalytische Propanoxidehydrierung auch kombiniert angewendet werden. Erfindungsgemäß wird das Propen und nicht umgesetztes Propan enthaltende Propanoxidehydrierproduktgasgemisch (in der Regel enthält es als weitere Bestandteile CO₂, CO, H₂O, N₂, O₂, Ethan, Ethen, Methan, Acrolein, Acrylsäure, Ethylenoxid, Butan, Essigsäure, Formaldehyd, Ameisensäure, Propylenoxid und Buten) unmittelbar in wenigstens eine weitere Reaktionszone geleitet, um das darin enthaltene Propen einer katalytischen Gasphasenoxidation zu Acrolein und/oder Acrylsäure zu unterwerfen.

Die vorgenannte gasphasenkatalytische Propanoxidation kann z.B. so durchgeführt werden, wie es im Stand der Technik, z.B. in der WO 97/36849 oder der CN-A 1105352 beschrieben ist. Selbstverständlich kann diese gasphasenkatalytische Propenoxidation auch wie in der EP-A 117146, der US-A 5198578 oder der US-A 5183936 beschrieben erfolgen. Sie kann aber auch in Analogie zur DE-A 3313573, CA-A 1217502, US-A 3161670 oder US-A 4532365 durchgeführt werden.

Dabei kann die gasphasenkatalytisch oxidative Umsetzung des im Propanoxidehydrierproduktgasgemisch enthaltenen Propen zu Acrolein und/oder Acrylsäure z.B. in einer oder in zwei nachfolgenden Oxidationszonen erfolgen. Das das Propen begleitende nicht umgesetzte Propan sowie neben N₂ und H₂O gegebenenfalls noch enthaltenes Edelgas, CO, CO₂ und andere niedere organische Verbindungen wie z.B. andere niedere Kohlenwasserstoffe fungieren dabei im wesentlichen als inertes Verdünnungsgas. Für den Fall, daß Acrylsäure das gewünschte Zielprodukt ist, werden sich in der Regel zwei gasphasenkatalytische Oxidationszonen an die Oxidehydrierung anschließen, wenngleich im Stand der Technik auch einstufige gasphasenkatalytische Oxidationen von Propen zu Acrylsäure bekannt sind. Ist Acrolein das gewünschte Zielprodukt, wird sich in der Regel nur eine gasphasenkatalytische Oxidationszone anschließen. Die vorgenannten katalytischen Gasphasenoxidationszonen können erfindungsgemäß z.B. in separaten Reaktoren realisiert werden.

D.h., die katalytische Gasphasenoxidation des im Propanoxidehydrierproduktgasgemisch enthaltenen Propens zu einer gegenüber Acrylsäure überwiegenden Menge an Acrolein kann z.B. wie in der EP-A 731 082, der DE-A 44 31 957, der DE-A 29 09 597 oder der EP-A 575 897 beschrieben durchgeführt werden.

D.h., die Gasphasenoxidation erfolgt in zweckmäßiger Weise in einem Vielkontaktrohr-Festbettreaktor.

In der Regel wird bei einem Propen : Sauerstoff : im wesentlichen indifferente Gase Volumen (N1)-Verhältnis von 1 : (1,0 bis 3,0) : (5 bis 25), vorzugsweise 1 : (1,7 bis 2,3) : (10 bis 15), gearbeitet.

Zum Erreichen der vorgenannten Verhältnisse ist es gegebenenfalls erforderlich, dem das Propen enthaltenden Propanoxidehydrierproduktgasgemisch vor seinem Einleiten in die Propenoxidationsstufe zusätzlich molekularen Sauerstoff zuzufügen. Dies kann in Form von Luft, in Form von an Stickstoff entreicherter Luft oder auch in Gestalt von reinem Sauerstoff erfolgen. Selbstverständlich können an dieser Stelle nach Belieben zusätzliche, im wesentlichen als indifferent bekannte, Verdünnungsgase zugesetzt. werden. Die Reaktionstemperatur wird zweckmäßigerweise zu 300°C bis 450°C, vorzugsweise zu 320°C bis 390°C gewählt. Der Reaktionsdruck beträgt üblicherweise 0,5x10⁵ - 5x10⁵ Pa (0,5 bis 5 bar), vorzugsweise 1x10⁵ - 3x10⁵ Pa (1 bis 3 bar). Die Gesamtraumbelastung beträgt häufig 1500 bis 2500 Nl/l/h.

Als Katalysatoren eignen sich für diese Oxidationsstufe z.B. diejenigen der DE-A 29 09 592, vor allem diejenigen aus dem Beispiel 1 dieser Schrift. Alternativ dazu können aber auch die Multimetalloxidkatalysatoren II bzw. II' der DE-A 1 97 53 817 eingesetzt werden. Dies trifft insbesondere auf die in diesen Schriften aufgeführten beispielhaften Ausführungsformen zu. Vor allem dann, wenn sie als Hohlzylindervollkatalysatoren wie in der EP-A 575 897 beschrieben gestaltet sind. Selbstverständlich kann in der Propenoxidationsstufe auch der Bi, Mo und Fe enthaltende Multimetalloxidkatalysator ACF-2 der Fa. Nippon Shokubai eingesetzt werden.

In der vorgenannten Propenoxidationsstufe wird kein reines Acrolein, sondern ein Gemisch erhalten, von dessen Nebenkomponenten das Acrolein in an sich bekannter Weise abgetrennt werden kann. Das so abgetrennte Acrolein kann als Zwischenprodukt zur Synthese verschiedener Endprodukte eingesetzt werden. Selbstverständlich kann es auch zur gasphasenkatalytischen Oxidation zur Herstellung von Acrylsäure eingesetzt werden. Bei einer erfindungsgemäßen Weiterverwendung des Acroleins zur Herstellung von Acrylsäure in einer weiteren gasphasenkatalytischen Oxidationszone werden die das Acrolein enthaltenden Reaktionsgase der Propenoxidationszone ohne Abtrennung von Nebenkomponenten in diese weitere Oxidationszone überführt. Gegebenenfalls durchlaufen sie zuvor eine Zwischenkühlung.

Diese weitere Oxidationszone kann in zweckmäßiger Weise ebenfalls in einem separaten Vielkontaktrohr-Festbettreaktor realisiert werden, wie es z.B. in der DE-A 44 31 949, der DE-A 44 42 346, der DE-A 1 97 36 105 oder der EP-A 731 082 beschrieben ist.

In der Regel wird dabei bei einem Acrolein : Sauerstoff : Wasserdampf : sonstige im wesentlichen indifferente Gase Volumen (Nl)-Verhältnis von 1 : (1 bis 3) : (> 0 bis 20) : (3 bis 30), vorzugsweise von 1 : (1 bis 3) : (0,5 bis 10) : (7 bis 18) gearbeitet. Zum Erreichen der vorgenannten Verhältnisse ist es gegebenenfalls erforderlich, dem Acrolein enthaltenden Produktgasgemisch aus der Propenoxidationszone vor seinem Einleiten in die Acroleinoxidationszone zusätzlich molekularen Sauerstoff zuzufügen. Dies kann in Form von Luft, in Form von an Stickstoff entreicherter Luft oder auch in Gestalt von reinem Sauerstoff erfolgen. Selbstverständlich können an dieser Stelle nach Belieben zusätzliche, im wesentlichen als indifferent bekannte Verdünnungsgase zugesetzt werden. Die Reaktionstemperatur wird zweckmäßigerweise zu 200°C bis 300°C, vorzugsweise zu 220 bis 290°C gewählt. Der Reaktionsdruck beträgt üblicherweise 0,5x10⁵ - 5x10⁵ Pa (0,5 bis 5 bar), vorzugsweise 1x10⁵ - 3x10⁵ Pa (1 bis 3 bar). Die Gesamtraumbelastung beträgt vorzugsweise 1000 bis 2500 Nl/l/h. Als Katalysatoren eignen sich für diese Oxidationsstufe z.B. diejenigen der allgemeinen Formel I bzw. I' aus der DE-A 44 42 346. Alternativ dazu können aber auch die Multimetalloxidkatalysatoren der DE-A 1 97 36 105, insbesondere die in dieser Schrift genannten Ausführungsbeispiele eingesetzt werden. Selbstverständlich kann auch der Bi, Mo und Fe umfassende Multimetalloxidkatalysator ACS-4 der Fa. Nippon Shokubai in der Acroleinoxidationsstufe eingesetzt werden.

Erfindungsgemäß wesentlich ist, daß die verschiedenen vorstehend beschriebenen Reaktionszonen auch in einem einzigen Reaktor verwirklicht werden können, wie es z.B. in der DE-A 19807079 beschrieben ist. D.h., die für eine katalytische Oxidehydrierung und die nachfolgende Propen-/Acroleinoxidation erforderlichen Katalysatoren können z.B. innerhalb ein und desselben Reaktionsrohres als zwei bzw. drei aufeinanderfolgende Katalysatorschüttungen (drei aufeinanderfolgenden Reaktionszonen) realisiert werden. Soll die Oxidehydrierung verwirklicht werden, kann in vorgenannter Anordnung die Oxidehydrierkatalysatorschüttung z.B. durch eine Leerstrecke im Reaktionsrohr als die entsprechende Reaktionszone ersetzt werden. Dabei können die einzelnen Katalysatorschüttungen und die Leerstrecke bei ein und derselben Temperatur oder auch mit einem temperaturstrukturierten(Mehrzonen-)reaktor bei unterschiedlichen Temperaturen betrieben werden.

Das die Acroleinoxidationszone verlassende Gasgemisch besteht selbstredend nicht aus reiner Acrylsäure, sondern aus einem letztere enthaltenden Gasgemisch, aus welchem Acrylsäure in an sich bekannter Weise abgetrennt werden kann.

Die verschiedenen bekannten Varianten der Acrylsäureabtrennung sind z.B. in der DE-A 1 96 00 955 zusammenfassend dargestellt. In entsprechender Weise könnte auch das Acrolein aus dem die Propenoxidationszone verlassenden Reaktionsgasgemisch abgetrennt werden. Gemeinsames Merkmal der Trennverfahren ist, daß das gewünschte Produkt entweder durch Absorption mit einem Lösungsmittel (vgl. auch DE-A 43 08 087) oder durch Absorption mit Wasser und/oder durch partielle Kondensation aus dem Reaktionsgasgemisch der Acroleinoxidationsstufe abgetrennt wird (das dabei resultierende Absorbat bzw. Kondensat wird anschließend destillativ (gegebenenfalls unter Zusatz eines azeotropen Schleppmittels) und/oder kristallisativ aufgearbeitet und so im wesentlichen reine Acrylsäure bzw. reines Acrolein gewonnen).

Die Trennlinie wird dabei im wesentlichen in allen Fällen so gezogen, daß ein im wesentlichen an Acrylsäure und/oder Acrolein sowie größtenteils an H₂O freier (in der Regel beträgt der H₂O-Volumenanteil des Restgasstromes ≤ 10 Vol.-%) Restgasstrom entsteht, dessen Hauptbestandteile N₂ und nicht umgesetztes Propan sind. Zusätzlich kann der Restgasstrom z.B. geringe Mengen an Gasen wie Kohlenoxide (CO, CO₂), Edelgase, O₂, H₂O und nicht umgesetztes Propen enthalten. Die vor der Weiterverwendung des vorgenannten Restgasstromes als Kreisgas erfindungsgemäß erforderliche Abtrennung wenigstens eines Teils des darin enthaltenen Luftstickstoffs kann z.B. in einfacher Weise destillativ erfolgen (dabei ist es selbstredend möglich, die Stickstoffabtrennung nur bei einer, z.B. 50 bis 70 % betragenden, Teilmenge des Restgasstromes vorzunehmen).

Zweckmäßigerweise wendet man eine fraktionierte Destillation an; vorzugsweise eine fraktionierte Druckdestillation bei tiefen Temperaturen. Der anzuwendende Druck kann z.B. 10 bis 100 bar betragen. Als Rektifikationskolonnen können Füllkörperkolonnen, Bodenkolonnen oder Packungskolonnen eingesetzt werden. Als Bodenkolonnen eignen sich solche mit Dual-Flow-Böden, Glockenböden oder Ventilböden. Das Rücklaufverhältnis kann z.B. 1 bis 10 betragen. Andere Möglichkeiten der Stickstoffabtrennung sind z.B. Druckwechselabsorption, Druckwäsche und Druckextraktion.

Bezogen auf die im Restgasstrom enthaltene Gesamtmenge an Stickstoff kann die erfindungsgemäß abzutrennende Stickstoffmenge 5 %, oder 10 %, oder 20 %, oder 30 %, oder 40 %, oder 50 %, oder 60 %, oder 70 %, oder 80 %, oder 90 % oder 95 bis 100 % betragen.

Selbstredend ist es auch möglich, den Stickstoff nicht für sich vom Restgasstrom abzutrennen, sondern gemeinsam mit anderen, im Rahmen einer kontinuierlichen Durchführung des erfindungsgemäßen Verfahrens wenig erwünschten Bestandteilen des rückzuführenden Restgasstromes. Beispielsweise kann man die Trennlinie bei einer fraktionierten destillativen Abtrennung des Stickstoffs vom Restgasstrom so legen, daß z.B. am Kopf der Rektifikationskolonne im wesentlichen alle diejenigen Bestandteile abgetrennt werden, deren Siedepunkt tiefer als der Siedepunkt von Propen liegt. Diese Bestandteile werden in erster Linie die Kohlenoxide CO und CO₂ sowie nicht umgesetzter Sauerstoff und Ethylen sowie Methan sein. Selbstverständlich kann gemeinsam mit dem Stickstoff auch nur ein Teil der vorgenannten Bestandteile abgetrennt werden. Vorzugsweise wird das erfindungsgemäße Verfahren so durchgeführt, daß zu dem Zeitpunkt zu dem das Produktgasgemisch die letzte Reaktionszone verläßt, wenigstens 70 %, vorzugsweise wenigstens 80 %, des den verschiedenen Reaktionszonen insgesamt zugeführten molekularen Sauerstoff umgesetzt worden sind.

### Beispiele

A) Herstellung von Acrolein und/oder Acrylsäure ausgehend von Propan (im Rahmen der nachfolgend angegebenen Analytik wird der geringe in Luft enthaltene Anteil an Edelgasen dem Stickstoffanteil der Luft zugerechnet)
   99,5 mol/h Luft und 74,9 mol/h Kreisgas der Zusammensetzung
   92,2 Vol.-% Propan,
   0,4 Vol.-% Propen,
   6,8 Vol.-% H₂O,
   0,1 Vol.-% CO₂ und
   0,5 Vol.-% andere Komponenten,
   wurden zu 174,4 mol/h Reaktionsgasausgangsgemisch vereinigt, auf einen Druck von 2,2 bar verdichtet und auf eine Temperatur von 430°C erwärmt. Mit vorgenanntem Reaktionsgasausgangsgemisch wurde ein 3,8 m langes Reaktionsrohr aus V2A Stahl (2,0 mm Wandstärke; 2,6 cm Innendurchmesser) beschickt, das auf seiner gesamten Länge auf eine Temperatur von 430°C salzbadgekühlt wurde. In Strömungsrichtung war das Reaktionsrohr auf einer Länge von 0,8 m zunächst mit kugelförmigen (Durchmesser = 8 mm) Steatitformkörpern (anstelle der Steatitkugeln können hier auch Ringe der unten genannten Geometrie 5 mm X 3 mm x 2 mm eingesetzt werden) beschickt. Auf der restlichen Schüttlänge von 3 m war das Reaktionsrohr mit einer Schüttung des Multimetalloxidkatalysators gemäß Beispiel 1, a)/Multimetalloxidmasse I der DE-A 19753817, gepreßt zu Vollkatalysatorzylindern der Geometrie 5 mm x 3 mm x 2 mm (Außendurchmesser x Höhe x Innendurchmesser) , gefüllt. Der Eingangsdruck betrug 2x10⁵ Pa (2,0 bar), der Ausgangsdruck lag bei 1,3x10⁵ Pa (1,3 bar). Das das Reaktionsrohr verlassende Produktgemisch (181,8 mol/h) wies folgende Zusammensetzung auf:
   0,1 Vol.-% Acrolein,
   33,3 Vol.-% Propan,
   3,6 Vol.-% Propen,
   4,8 Vol.-% O₂,
   42,7 Vol.-% N₂,
   11,6 Vol.-% H₂O,
   2,0 Vol.-% CO,
   1,5 Vol.-% CO₂ und
   0,4 Vol.-% andere Komponenten.

   Dem vorgenannten auf 2,4 bar verdichteten Produktgemisch wurden 38,7 mol/h Luft zugemischt, wodurch für die nachfolgende gasphasenkatalytische Propenoxidation ein Reaktionsgasausgangsgemisch entstand, das nachfolgende Zusammensetzung aufwies:
   0,1 Vol.-% Acrolein,
   27,4 Vol.-% Propan,
   3,0 Vol.-% Propen,
   8,0 Vol.-% O₂,
   48,6 Vol.-% N₂,
   9,8 Vol.-% H₂O,
   1,6 Vol.-% CO,
   1,2 Vol.-% CO₂ und
   0,3 Vol.-% andere Komponenten.

   Ein Reaktionsrohr (V2A Stahl; Länge 3,80 m; 2,0 mm Wandstärke; 2,6 cm Innendurchmesser) wurde in Ausströmrichtung zunächst auf einer Länge von 50 cm mit einer Vorschüttung aus Steatit-Kugeln (Durchmesser: 4-5 mm) beschickt. Auf einer Kontaktrohrlänge von 3,00 m schloß sich eine Schüttung des Multimetalloxidkatalysators gemäß Beispiel 1, 3./Multimetalloxid II aus der DE-A 19753817 an. Das Reaktionsrohr wurde auf seiner gesamten Länge mit einem Salzbad auf 350°C gehalten und mit 220,5 mol/h des vorgenannten Reaktionsgasausgangsgemisches (das eine Temperatur von 200°C aufwies) beschickt. Der Ausgangsdruck betrug 1,9x10⁵ Pa (1,9 bar).
   Dem das Reaktionsrohr der Propenoxidationsstufe verlassenden Produktgasgemisch wurden 5,4 mol/h Luft zugemischt und mit dem dabei entstehenden Reaktionsausgangsgemisch, das auf eine Temperatur von 200°C gebracht worden war, wurde das nachfolgend beschriebene Acroleinoxidationsrohr beschickt.
   Dieses Reaktionsrohr (V2A Stahl; Länge: 3,80 m, 2,0 mm Wandstärke; 2,6 cm Innendurchmesser) war in Ausströmrichtung zunächst auf einer Länge von 50 cm mit einer Vorschüttung aus Steatit-Kugeln (Durchmesser: 4-5 mm) beschickt. Auf einer Kontaktrohrlänge von 2,70 m schloß sich eine Schüttung des Multimetalloxidkatalysators gemäß Beispiel b, S1 der DE-A 4442346 an. Das Reaktionsrohr wurde auf seiner gesamten Länge mit einem Salzbad auf 270°C gehalten und mit dem vorstehend beschriebenen Reaktionsgasausgangsgemisch beschickt. Der Eingangsdruck lag bei 1,8x10⁵ Pa (1,8 bar) und der Ausgangsdruck betrug 1,3x10⁵ Pa (1,3 bar).
   Das das Reaktionsrohr in einer Menge von 223,7 mol/h verlassende Produktgemisch wies nachfolgende Zusammensetzung auf:
   2,5 Vol.-% Acrylsäure,
   0,1 Vol.-% Essigsäure,
   27,0 Vol.-% Propan,
   3,0 Vol.-% O₂,
   50,2 Vol.-% N₂,
   13,1 Vol.-% H₂O,
   1,9 Vol.-% CO,
   1,9 Vol.-% CO₂ und
   0,3 Vol.-% andere Komponenten.

   Das die Acroleinoxidationsstufe verlassende heiße Reaktionsgas wurde in einem Venturiwäscher (Quenchapparat) durch direkten Kontakt mit durch im Bereich des engsten Querschnitts des Venturi-Rohres angebrachte Schlitze eingedüster Quenchflüssigkeit (140-150°C) aus 57,4 Gew.-% Diphenylether, 20,7 Gew.-% Diphenyl und 20 Gew.-% o-Dimethylphthalat auf eine Temperatur von ca. 160°C abgekühlt. Anschließend wurde in einem nachgeschalteten Tropfenabscheider (Vorlagebehälter mit oben weggeführtem Gasrohr) der tropfenförmig flüssig gebliebene Anteil der Quenchflüssigkeit von der aus Reaktionsgas und verdampfter Quenchflüssigkeit bestehenden Gasphase abgetrennt und in einem Kreislauf I zum Venturiwäscher rückgeführt. Ein Teilstrom der rückgeführten Quenchflüssigkeit wurde dabei einer Lösungsmitteldestillation unterzogen, wobei die Quenchflüssigkeit überdestilliert wurde und schwersiedende Nebenkomponenten, die verbrannt wurden, zurückblieben. Die überdestillierte Quenschflüssigkeit wurde dem Ablauf der nachfolgend beschriebenen Absorptionskolonne zugeführt.
   Die eine Temperatur von ca. 160°C aufweisende Gasphase wurde in den unteren Teil einer Füllkörperabsorptionskolonne geführt (3 m hoch; Doppelmantel aus Glas; Innendurchmesser 50 mm; drei Füllkörperzonen der Längen (von unten nach oben) 90 cm, 90 cm und 50 cm; die Füllkörperzonen waren von unten nach oben wie folgt thermostatisiert: 90°C, 60°C, 20°C; die vorletzte und die letzte Füllkörperzone waren durch einen Kaminboden getrennt; die Füllkörper waren Metallwendeln aus Edelstahl mit einem Wendeldurchmesser von 5 mm und einer Wendellänge von 5 mm; unmittelbar oberhalb der mittleren Füllkörperzone wurde das Absorptionsmittel zugeführt) und dem Gegenstrom von 2400 g/h des gleichfalls aus 57,4 Gew.-% Diphenylether, 20,7 Gew.-% Diphenyl und 20 Gew.-% o-Dimethylphthalat zusammengesetzten, mit einer Temperatur von 50°C aufgegebenen Absorptionsmittels ausgesetzt. Der Ablauf der Absorptionskolonne, der neben Acrylsäure auch leichsiedende Nebenprodukte wie z.B. Acrolein und Essigsäure absorbiert enthielt, wurde in einem Wärmetauscher indirekt auf 100°C erwärmt und auf den Kopf einer Desorptionskolonne gegeben, die ebenfalls als Füllkörperkolonne einer Länge von 2 m ausgeführt war (Doppelmantel aus Glas; 50 mm Innendurchmesser; Füllkörper: Edelstahlwendeln mit einem Wendeldurchmesser von 5 mm und einer Wendellänge von 5 mm; eine Füllkörperzone der Länge 1 m; thermostatisiert auf 120°C). In der Desorptionskolonne wurden die im Vergleich zur Acrylsäure leichtersiedenden Komponenten wie Acrolein und Essigsäure durch Strippen mit 9,1 mol/h Propan (Gegenstrom; Zuführtemperatur 120°C) weitgehend aus dem Acrylsäure/Absorptionsmittel-Gemisch entfernt. Das die Desorptionskolonne verlassende beladene Propan (Strippgas) wurde rezirkuliert und mit dem heißen Reaktionsgas der Acroleinoxidationsstufe vor dessen Eintritt in den Venturiquensch vereinigt.
   Das in der Absorptionskolonne die zweite Füllkörperzone nach oben verlassende nicht absorbierte Gasgemisch wurde in der dritten Füllkörperzone weiter abgekühlt, um den leicht kondensierbaren Teil der darin enthaltenen Nebenkomponenten, z.B. Wasser und Essigsäure, durch Kondensation abzutrennen. Dieses Kondensat wird Sauerwasser genannt. Zur Erhöhung der Trennwirkung wurde ein Teil des Sauerwassers oberhalb der dritten Füllkörperzone der Absorptionskolonne mit einer Temperatur von 20°C in die Absorptionskolonne rückgeführt. Die Entnahme des Sauerwassers erfolgte unterhalb der obersten Füllkörperzone vom dort angebrachten Kaminboden. Das Rücklaufverhältnis betrug 200. Die kontinuierlich entnommene Sauerwassermenge betrug 24,4 mol/h. Sie enthielt neben 94,3 Gew.-% Wasser auch noch 3,0 Gew.-% Acrylsäure. Diese kann bei Bedarf wie in der DE-A 1 96 00 955 beschrieben rückgewonnen werden. Der die Absorptionskolonne letztlich verlassende Gasstrom bildete den Restgasstrom.
   Die Sumpfflüssigkeit der Desorptionskolonne wurde auf dem 8ten Boden von unten einer 57 Dual-Flow-Böden enthaltenden Bodenkolonne zugeführt (Innendurchmesser: 50 mm; Länge 3,8 m; Kopfdruck: 1x10⁴ Pa (100 mbar); Sumpfdruck: 2,8x10⁹ Pa (280 mbar): Sumpftemperatur: 195°C; auf dem 9ten Boden war ein Druckverlustwiderstand angebracht;) und in selbiger rektifiziert. Vom 48ten Boden von unten wurden je Stunde 5,4 mol einer Roh-Acrylsäure via Seitenabzug entnommen. Die Reinheit der entnommenen Roh-Acrylsäure lag bei ≥ 98 Gew.-%. Am Kopf der Rektifikationskolonne wurde nach einer Partialkondensation (Rücklaufverhältnis: 8,7) ein an Leichtsiedern angereicherter, Acrylsäure enthaltender, Gasstrom abgezogen, der oberhalb der untersten Füllkörperzone in die Absorptionskolonne rückgeführt wurde. Aus dem Sumpf der Rektifikationskolonne wurde das an Leichtsiedern freie und an Acrylsäure nahezu freie Absorptionsmittel abgezogen und oberhalb der zweiten Füllkörperzone (von unten betrachtet) in die Absorptionskolonne rückgeführt. Dem Rücklauf am Kopf der Rektifikationskolonne wurde Phenothiazin als Polymerisationsinhibitor zugesetzt und zwar in solchen Mengen, daß der Seitenabzug 300 ppm Phenothiazin enthielt (eine schematische Darstellung des Aufarbeitungsverfahrens des Reaktionsgases der Acroleinoxidationsstufe zeigt die DE-A 1 96 00 955; darüber hinaus ist die Aufarbeitungsweise auch in der DE-A 4308087 dargestellt).
   Der die Absorptionskolonne verlassende Restgasstrom wies nachfolgende Zusammensetzung auf:
   34,2 Vol.-% Propan,
   3,3 Vol.-% O₂,
   55,4 Vol.-% N₂,
   2,5 Vol.-% H₂O,
   2,1 Vol.-% CO,
   2,1 Vol.-% CO₂ und
   0,4 Vol.-% andere Komponenten.

   Seine Menge betrug 203,0 mol/h. Er wurde auf 6x10⁶ Pa (60 bar) verdichtet, auf 70°C abgekühlt und dann einer unter Druck betriebenen Rektifikationskolonne zugeführt, die 51 Böden aufwies. Die Zufuhr des Restgasstromes erfolgte auf dem 30. Boden von unten. Der Kopfdruck der Rektifikationskolonne (einer Glocken-Bodenkolonne, Durchmesser 50 mm) betrug 60 bar. Der Kolonnenkopf wurde mit dem Kühlmittel Baysilone® KT3 gekühlt (Vorlauftemperatur - 50°C). Die Sumpf temperatur lag bei 92°C. Im Kolonnensumpf wurde ein Teil der entnommenen Sumpfflüssigkeit als Siededampf rückgeführt.
   Am Kolonnenkopf wurden so 128,2 mol/h eines Abgases abgeführt, das nachfolgende Zusammensetzung aufwies:
   87,7 Vol.-% N₂,
   3,3 Vol.-% CO,
   0,4 Vol.-% Propan,
   5,2 Vol.-% O₂,
   3,2 Vol.-% CO₂ und
   0,2 Vol.-% andere Komponenten.

   Die kontinuierlich entnommene Sumpfflüssigkeit betrug 74,9 mol/h. Sie wurde in die Dampfphase überführt und wie eingangs beschrieben als Kreisgas an die katalytische Propanoxidehydrierung rückgeführt.
   Bezogen auf die eingesetzte Propanmenge betrug die Ausbeute an Acrylsäure 59,3 mol-% und die Selektivität der Acrylsäurebildung betrug 60,5 mol.-%. Der Sauerstoffumsatz, bezogen auf die insgesamt eingesetzte Sauerstoffmenge, lag bei 79,8 mol.-%.
   Abschließend sei vermerkt, daß erfindungsgemäß anstelle von Propan auch Kreisgas zum Strippen eingesetzt werden kann. In diesem Fall kann man das erforderliche Propan z.B. direkt der Oxidehydrierungsstufe zuführen.
B) Herstellung von 1 mol/s an Acrylsäure (20°C, 1x10⁵ Pa (1 bar)) durch Kombination von Propanoxidehydrierung und katalytischer Gasphasenoxidation - Betrachtung der zur Stickstoffabtrennung zu leistenden Trennarbeit (zur Vereinfachung der Betrachtung wird hier ein 50 %iger Umsatz des Propans bei der Oxidehydrierung und ein vollständiger Umsatz bei der Gasphasenoxidation sowie eine 100 %ige Selektivität der jeweiligen Zielverbindung angenommen).

### a) Verwendung von reinem O₂ als Sauerstoffquelle

Die Reaktionsstöchiometrie der Oxidehydrierung von Propan zu Propen lautet:

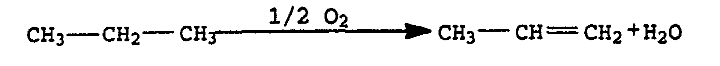

Die Reaktionsstöchionmetrie der katalytischen Gasphasenoxidation von Propen zu Acrylsäure lautet:

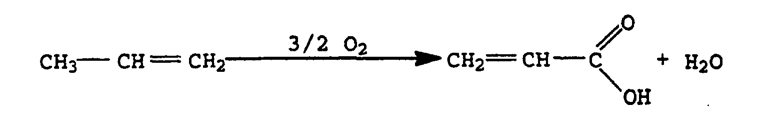

Demnach werden zur Erzeugung von 1 mol/s Acrylsäure durch Oxidehydrierung von Propan und anschließende Gasphasenoxidation von Propen (unter den oben genannten Bedingungen) 2.0 mol/s O₂ benötigt.

Ausgangsmaterial zur Gewinnung von 2,0 mol/s O₂ ist Luft (78 Vol.-% N₂, 21 Vol.-% O₂ und 1 Vol.-% Restgase), deren Zusammensetzung der Einfachheit halber zu 80 Vol.-% N₂ und 20 Vol.-% O₂ angenommen werden soll. Ferner soll ideales Verhalten angenommen werden. Zur Gewinnung der 2,0 mol/s kann demnach von 10,0 mol/s Luft (=Gemisch aus 8,0 mol/s N₂ und 2,0 mol/s O₂) ausgegangen werden, die eine Temperatur von 20°C und einen Druck von 1x10⁵ Pa (1 bar) aufweist.

Um aus vorgenannten 10,0 mol/s Luft 2,0 mol/s reinen O₂ abzutrennen, wird die Luft unter Aufrechterhaltung des Druckes von 1 bar auf -194°C abgekühlt und als in siedendem Zustand befindliche Flüssigkeit in den Mittelteil einer bei 1x10⁵ Pa (1 bar) adiabat betriebenen Rektifikationskolonne geführt, um über Kopf in reinen Stickstoff und als Sumpfflüssigkeit in reinen Sauerstoff aufgetrennt zu werden.

Um diese geforderte Gemischzerlegung zu verwirklichen ist gemäß "K. Sattler, Thermische Trennverfahren, Grundlagen, Auslegung, Apparate, Zweite Auflage, Verlag Chemie, Weinheim (1995), S. 182" bei Verwendung einer idealen, d.h., unendlich viele Trennstufen aufweisenden, Rektifiktionskolonne am Kolonnenkopf ein Mindestrücklaufverhältnis erforderlich (dabei gilt: ja größer das erforderliche Mindestrücklaufverhältnis, desto größer die zu leistende Trennarbeit).

Während das Rücklaufverhältnis am Kolonnenkopf beim gegebenen Trennproblem ganz allgemein definiert ist als das Verhältnis des Anteils der am Kopf der Rektifikationskolonne je Zeiteinheit gasförmig anfallenden N₂-Menge, der nach Kondensation in die Rektifikationskolonne rückgeführt wird, zu demjenigen Anteil der entnommen wird, ist das Mindestrücklaufverhältnis am Kolonnenkopf (νₘᵢₙ) definiert als das Verhältnis des Anteils der am Kopf der Rektifikationskolonne je Zeiteinheit gasförmig anfallenden N₂-Menge, der zur Verwirklichung der gestellten Trennaufgabe nach Kondensation mindestens in die Rektifikationskolonne zurückgeführt werden muß, zu demjenigen Anteil, der entnommen wird. Gemäß vorgenanntem Zitat nach K. Sattler gilt: 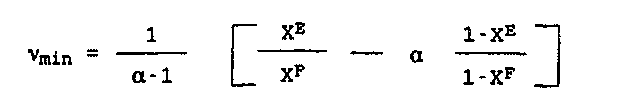 mit
- α=: relative Flüchtigkeit oder Trennfaktor von N₂ und O₂ (d.h. = Verhältnis der Sättigungsdampfdrücke von N₂ und O₂ bei der mittleren Trenntemperatur längs der Rektifikationskolonne (die Trennung bzw. Gemischzerlegung vollzieht sich längs der gesamten Rektifikationskolonne)).

Die Siedetemperatur von N₂ beträgt bei einem Druck von 1 bar = -196°C (=Kopftemperatur). Die Siedetemperatur von O₂ beträgt bei einem Druck von 1x10⁵ Pa (1 bar) = -183°C (=Sumpftemperatur). Damit beträgt die mittlere Siedetemperatur längs der Rektifikationskolonne = -190°C. Gemäß VDI-Wärmeatlas, 5. Auflage 1998 (DC6 und DC7) beträgt der Sättigungsdampfdruck von N₂ bei -190°C = 2x10⁵ Pa (2,0 bar) und der Sättigungsdampfdruck von O₂ bei -190°C = 4,8x10⁴ Pa (0,48 bar). Damit ergibt sich das relevante α zu 2,0 : 0,48 = 4,15.

X^{F} ist der N₂-Molenbruch im der Rektifikationskolonne zugeführten, zu trennenden, Gemisch (d.h. in Luft). D.h., X^{F} = 0,8 (=̂ 80 Vol.-% N₂ in Luft).

X^{E} ist der N₂-Molenbruch im kopfabzug. Da am Kopf reiner Stickstoff abgetrennt werden soll, gilt X^{E} = 1. Somit rechnet sich νₘᵢₙ zu 0,4. D.h., um 8 mol/s reinen N₂ am Kolonnenkopf entnehmen zu können, müssen 3,2 mol/s N₂ in die Kolonne rückgeführt werden. Insgesamt werden der Kolonne somit je Zeiteinheit 11,2 mol/s an flüssigem N₂ zugeführt (8,0 mol/s als Bestandteil der flüssigen Luft und 3,2 mol/s als Rücklauf am Kolonnenkopf), deren Gesamtmenge kontinuierlich verdampft werden muß. Um diese Flüssigkeitsmenge zu verdampfen, muß in den Kolonnensumpf eine entsprechende Flüssigkeitsmenge als Dampf rückgeführt werden. Gleichzeitig werden am Kolonnensumpf 2,0 mol/s an O₂ entnommen.

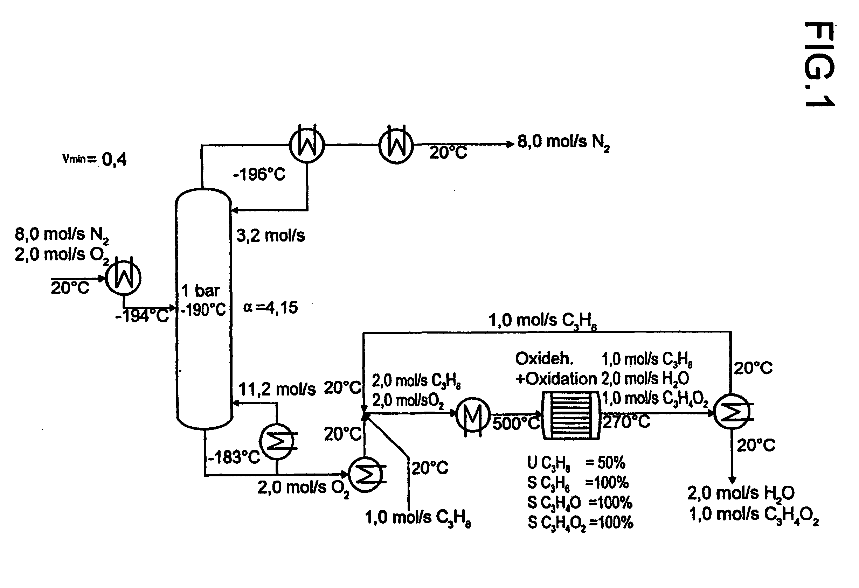

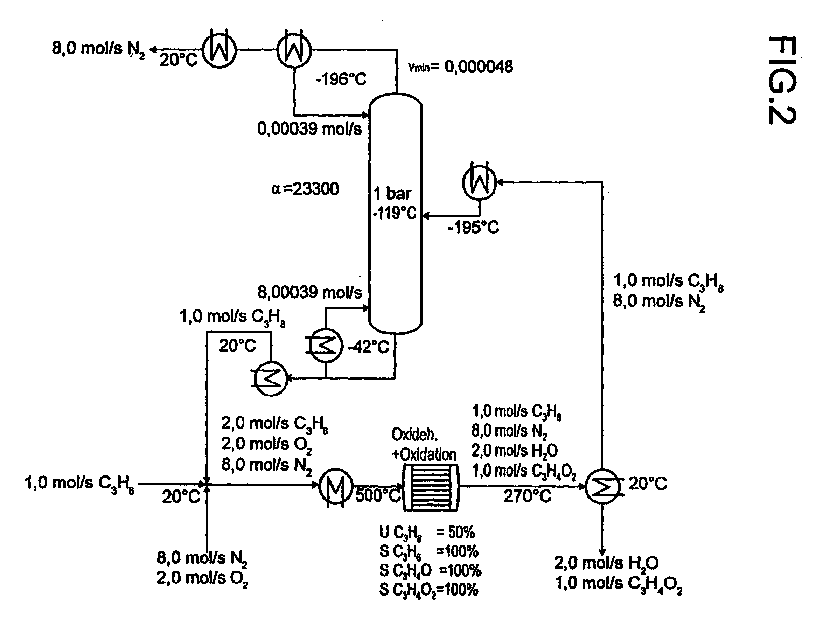

Der am Kolonnensumpf flüssig entnommene Sauerstoff wird bei 1 bar auf 20°C erwärmt, mit 2 mol/s Propan (20°C, 1 bar) vermischt, unter Beibehalt des Druckes von 1 bar auf 500°C erwärmt und oxidehydriert (entweder katalytisch und/oder homogen) sowie danach gasphasenkatalytisch oxidiert. Das aus I mol/s Propen, 2 mol/s H₂O und 1,0 mol/s Acrylsäure bestehende Reaktionsgasgemisch wird anschließend unter Beibehalt des Druckes auf 20°C abgekühlt, wobei der Wasserdampf und die Acrylsäure infolge der hohen Siedetemperaturdifferenz vollständig auskondensieren und 1 mol/s reines Propan 1x10⁵ Pa (1 bar), 20°C) erhalten wird, das zur Oxidehydrierung rückgeführt wird. Eine Übersicht des Vorstehenden zeigt die Figur 1.

### b) Verwendung von Luft als Sauerstoffquelle

Ein Gemisch aus 10,0 mol/s Luft (2,0 mol/s O₂ und 8,0 mol/s N₂) und 2 mol/s Propan, das einen Druck von 1x10⁵ Pa (1 bar) und eine Temperatur von 20°C aufweist, wird unter Aufrechterhaltung des Druckes auf 500°C erwärmt und oxidehydriert (entweder katalytisch und/oder homogen) sowie danach gasphasenkatalytisch oxidiert. Das aus 1,0 mol/s Propan, 8,0 mol/s N₂, 2,0 mol/s H₂O und 1,0 mol/s Acrylsäure bestehende Reaktionsgasgemisch wird anschließend unter Beibehalt des Druckes auf 20°C abgekühlt, wobei der Wasserdampf und die Acrylsäure infolge der hohen Siedetemperaturdifferenz vollständig auskondensieren und 1,0 mol/s Propan und 8,0 mol/s N₂ einen Restgasstrom bilden.

Dieses Gasgemisch wird unter Druckerhalt soweit abgekühlt (-195°C), daß es als siedende Flüssigkeit in den Mittelteil einer unendlich viele theoretische Trennstufen aufweisenden, bei einem Druck von 1 bar adiabat betriebenen, Rektifikationskolonne geführt werden kann, um über Kopf in reinen Stickstoff und als Sumpfflüssigkeit in reines Propan aufgetrennt zu werden.

Das für die Trennung erforderliche Mindestrücklaufverhältnis am Kolonnenkopf berechnet sich in entsprechender Weise wie in a) zu νₘᵢₙ = 0,000048.

Die zur Berechnung von νₘᵢₙ erforderlichen Daten sind: Die Siedetemperatur von N₂ beträgt bei einem Druck von 1 bar =-196°C (= Kopftemperatur). Die Siedetemperatur von Propan beträgt bei einem Druck von 1 bar = -42°C (= Sumpftemperatur). Damit beträgt die mittlere Siedetemperatur längs der Rektifikationskolonne = -119°C. Gemäß VDI-Wärmeatlas, 5. Auflage 1998 (DC6 und DC7) beträgt der Sättigungsdampfdruck von N₂ bei -119°C = 1x10⁵ Pa (104,9 bar) und der Sättigungsdampfdruck von Propan bei -119°C = 450 Pa (0,0045 bar). Damit ergibt sich das relevante α zu 104,9 : 0,0045 = 23300.

X^{F}, der N₂-Molenbruch im der Rektifikationskolonne zugeführten, zu trennenden Gemisch, beträgt 8/9.

X^{E}, der N₂-Molenbruch im Kopfabzug, beträgt 1.

Der am Kolonnenkopf gasförmig entnommene, Siedetemperatur aufweisende, Stickstoff (8 mol/s) wird bei 1 bar auf 20°C erwärmt.

Die 1,0 mol/s an flüssigem Propan, die dem Kolonnensumpf kontinuierlich entnommen werden, werden bei 1 bar auf 20°C erwärmt und in die Oxidehydrierung zurückgeführt.

Vergleicht man die Wege a), b) miteinander, so weist das geringere νₘᵢₙ im Fall b) (νₘᵢₙ = 0,000048) sowie der signifikant größere Trennfaktor (α = 23300) die bei Verwendung von Luft als Sauerstoffquelle gegenüber Fall a) (νₘᵢₙ = 0,4; α = 4,15) geringere zu leistende Trennarbeit aus.

## Patentansprüche

1. Verfahren zur Herstellung von Acrolein und/oder Acrylsäure aus Propan, bei dem man das Propan in einer ersten Reaktionszone einer partiellen homogenen und/oder heterogen katalysierten Oxidehydrierung mit molekularem Sauerstoff zu Propen unterwirft und anschließend das in der ersten Reaktionszone gebildete, Propen enthaltende, Produktgasgemisch ohne Abtrennung eines Produktgasgemischbestandteils in wenigstens eine weitere Reaktionszone führt und in dieser wenigstens einen weiteren Reaktionszone das im Produktgasgemisch der ersten Reaktionszone enthaltene Propen in Begleitung aller Produktgasgemischbestandteile der ersten Reaktionszone einer gasphasenkatalytischen Oxidation zu Acrolein und/oder Acrylsäure unterwirft, aus dem Produktgasgemisch der gasphasenkatalytischen Oxidation darin enthaltenes Acrolein und/oder Acrylsäure sowie Wasser abtrennt und das im dabei verbleibenden Restgasstrom enthaltene nicht umgesetzte Propan als Bestandteil des Restgasstroms in die erste Reaktionszone zurückführt, **dadurch gekennzeichnet, daß** man dem der ersten Reaktionszone zugeführten Reaktionsgasausgangsgemisch den in der ersten Reaktionszone benötigten, von Kreissauerstoff verschiedenen, molekularen Sauerstoff in Form von Luft zusetzt und vor der Rückführung des Restgasstromes in die erste Reaktionszone wenigstens einen Teil des im Restgasstrom enthaltenen Luftstickstoff vom Restgasstrom abtrennt.

2. Verfahren nach Anspruch 1, **dadurch gekennzeichnet, daß** man vor der Rückführung des Restgasstromes in die erste Reaktionszone die Gesamtmenge des im Restgasstrom enthaltenen Luftstickstoff vom Restgasstrom abtrennt.

3. Verfahren nach Anspruch 1 oder 2, **dadurch gekennzeichnet, daß** die Stickstoffabtrennung durch fraktionierte Destillation vorgenommen wird.

4. Verfahren nach Anspruch 3, **dadurch gekennzeichnet, daß** mit der Abtrennung des Stickstoffs eine Abtrennung aller, leichter als Propen siedenden, Bestandteile des Restgasstromes vorgenommen wird.

## Claims

1. A process for the preparation of acrolein and/or acrylic acid from propane, in which, in a first reaction zone, the propane is subjected to a partial oxydehydrogenation with molecular oxygen under homogeneous and/or heterogeneous catalysis to give propene, and the propene-containing product gas mixture formed in the first reaction zone is fed, without separating off a component of the product gas mixture, into at least one further reaction zone and, in this at least one further reaction zone, the propene contained in the product gas mixture of the first reaction zone, together with all components of the product gas mixture of the first reaction zone, is subjected to a gas-phase catalytic oxidation to acrolein and/or acrylic acid, acrolein and/or acrylic acid and water contained in the product gas mixture of the gas-phase catalytic oxidation are separated from said mixture, and the unconverted propane contained in the remaining residual gas stream is recycled as a component of the residual gas stream to the first reaction zone, wherein the molecular oxygen required in the first reaction zone and differing from recycled oxygen is added in the form of air to the reaction gas starting mixture fed to the first reaction zone and, before the recycling of the residual gas stream to the first reaction zone, at least a part of the atmospheric nitrogen contained in the residual gas stream is separated from the residual gas stream.

2. A process as claimed in claim 1, wherein, before the recycling of the residual gas stream to the first reaction zone, the total amount of the atmospheric nitrogen contained in the residual gas stream is separated from the residual gas stream.

3. A process as claimed in claim 1 or 2, wherein nitrogen is separated off by fractional distillation.

4. A process as claimed in claim 3, wherein all components of the residual gas stream which have a boiling point lower than that of propene are separated off together with the nitrogen.

## Revendications

1. Procédé de préparation d'acroléine et / ou d'acide acrylique à partir de propane, dans lequel, dans une première zone de réaction, on soumet le propane à une oxydéshydrogénation à catalyse homogène et / ou hétérogène par de l'oxygène moléculaire pour former du propène et, ensuite, on envoie le mélange de produit gazeux contenant du propène et formé dans la première zone de réaction dans au moins une autre zone de réaction, sans séparation d'un constituant du mélange de produits gazeux et, dans cette au moins une autre zone de réaction, on soumet le propène contenu dans le mélange de produits gazeux de la première zone de réaction, en même temps que tous les constituants du mélange gazeux de produits de la première zone de réaction, à une oxydation catalytique en phase gazeuse pour obtenir de l'acroléine et / ou de l'acide acrylique, on sépare du mélange gazeux de produits de l'oxydation catalytique en phase gazeuse l'acroléine et / ou l'acide acrylique ainsi que l'eau qui y sont contenus, et on renvoie dans la première zone de réaction le propane non converti contenu dans le courant de gaz résiduel qui subsiste ainsi, en tant que constituant du courant de gaz résiduel, **caractérisé en ce que** l'on ajoute au mélange gazeux réactionnel de départ envoyé à la première zone de réaction l'oxygène moléculaire nécessaire dans la première zone de réaction, et différent de l'oxygène de circuit, sous forme d'air, et qu'avant de renvoyer le courant de gaz résiduel dans la première zone de réaction, on sépare du courant de gaz résiduel au moins une partie de l'azote de l'air contenu dans le courant de gaz résiduel.

2. Procédé selon la revendication 1, **caractérisé en ce que**, avant de renvoyer le courant de gaz résiduel dans la première zone de réaction, on sépare du courant de gaz résiduel au moins une partie de l'azote de l'air contenu dans le courant de gaz résiduel.

3. Procédé selon la revendication 1 ou 2, **caractérisé en ce que**, avant de renvoyer le courant de gaz résiduel dans la première zone de réaction, on sépare la totalité de l'azote de l'air contenu dans le courant de gaz résiduel par distillation fractionnée.

4. Procédé selon la revendication 3, **caractérisé en ce qu'**avec la séparation de l'azote, on entreprend une séparation de tous les constituants du courant de gaz résiduel plus légers que le propène.
